# EUROPEAN PATENT APPLICATION

(11) **EP 1 611 877 A1**
(43) Date of publication of application: **04.01.2006**
(21) Application number: 05398006.6
(22) Date of filing: 27.06.2005
(51) Int. Cl.: A61K 9/00, A61P 27/06

(54) **Method for preparing sustained-release therapeutic ophthalmic articles using compressed fluids for impregnation of drugs**

(30) Priority: 28.06.2004 PT 10315404
(71) Applicant: Universidade de Coimbra, 3004-531 Coimbra (PT); IBET - Instituto de Biologia Experimental e Tecnologica, 2781-901 Oeiras (PT)
(72) Inventor: Cipriano de Sousa, Herminio José, 3000-036 Coimbra (PT); Mendes Gil, Maria Helena, 3000-135 Coimbra (PT); Martins Duarte, Catarina Maria, 2910-008 Setúbal (PT); Baptista Leite, Eugönio Oscar, 3000-351 Coimbra (PT); Cruz Duarte, Ana Rita, Estrada das Areias No. 372, 3000-351 Estoril (PT)
(74) Representative: Pereira da Cruz, Joao

(57) **Abstract**

The present invention describes a method for the impregnation of a drug or a drug composition into ophthalmic articles, in order to prepare drug sustained release systems mainly for the treatment of glaucoma and other eye diseases. Ophthalmic articles can be for example contact lenses.

The drug or drug composition is dissolved in a compressed fluid, or mixture of compressed fluids, in a liquid, sub-critical liquid, gaseous or supercritical state. Co-solvents can be added to increase drug solubility in the compressed fluids. The mixture is subsequently contacted with the ophthalmic article. This process can be done in a single step or in a double step manner. The impregnation can be carried out in finished or semifinished ophthalmic articles.

## Description

### FIELD OF THE INVENTION

This invention refers to a new process to prepare therapeutic ophthalmic articles using compressed fluids. A drug, or a drug composition, is impregnated into ophthalmic articles with the aim of preparing drug sustained release systems mainly for the treatment of glaucoma and other eye diseases. Nevertheless, other human pathologies can also be treated with these drug sustained release systems.

### BACKGROUND OF THE INVENTION

Impregnation of polymers and copolymers can be performed by soaking the polymers or copolymers in the liquid additives to be impregnated, or in a liquid solution containing those additives dissolved. However, this method presents several disadvantages. Most polymers and copolymers are difficult to impregnate in this manner because of low diffusion of the additives and slow rates of impregnation obtained. Increasing the temperature can improve the process, but it can also degrade the polymers, copolymers and additives to be impregnated. Furthermore, when using a solution containing the additives to be dissolved, a final step of solvent removal is needed. Usually, this final step requires relatively high temperatures, which can also degrade the substances involved, and complete removal of this solvent from the polymer/additive system is very difficult, if not impossible, to carry out. This fact is of extreme importance when the solvents used are dangerous or toxic.

Polymer impregnation can also be done by mixing the additives with the polymers/copolymers and subsequently the resulting mixture is masticated, heated, mixed and melted before being, for example, injected into moulds. Also in this case, relatively high temperatures are required to melt the polymers and copolymers, which can degrade thermally labile additives and/or polymers or copolymers. It is also possible that, for certain polymers or copolymers, pre-curing will occur at relatively high temperatures.

Finally, the most used method for the impregnation of additives into polymer/copolymer matrix is the one which occurs during their own polymerization reactions. Additives to impregnate are initially mixed together with liquid monomers and co-monomers, solvents, initiators, etc. As the polymerization reaction proceeds, polymers and copolymers are formed and the additive to be impregnated is physically trapped by occlusion inside the solid polymer matrix.

In the particular case of finished or semi-finished transparent ophthalmic articles, there are several conventional methods to incorporate additives into transparent organic polymer matrices. These additives can be drugs or drug compositions, stabilizers, compatibilizing agents, plasticizers, antioxidants, antiozonants, biocides, UV absorbers, dyes and pigments, etc.

A first method consists in adding the substances to impregnate in the liquid reactive mixture containing the monomers, co-monomers, initiators, etc. Then, the resulting mixture is transferred into a mold (a two-sided or a one-sided mold) and polymerized by a thermal or photochemical route. The transparent ophthalmic article is then recovered after polymerization with the desired additives entrapped. Typical manufacturing methods are spincasting, lathe cutting, combination of lathe cutting and spincasting, dry and wet cast molding and lightstream mask technology. However, these methods present several disadvantages, such as long polymerization times and the use of large amounts of initiator. Furthermore, the above mentioned methods require thermal or photochemical polymerization reactions, with their respective disadvantages.

For example, if the reactive mixture contains dyes, pigments or any other UV absorbing substances, the polymerization reaction should be carried out thermally, at relatively high temperatures, because these additives can absorb light and thus damage or even prevent photochemical polymerization reactions. These additives can also react with radical initiators, acting as chain transfer agents, consequently inhibiting or preventing the polymerization reactions. Similarly, some drugs or other additives to be impregnated in this manner may also act in the same way and cause problems in polymerization reactions. However, even when photochemical polymerization is possible, relatively high temperatures are usually required which, together with the long polymerization times required, can sometimes irreversibly degrade the additives to impregnate and the polymers and copolymers involved.

Another method used to impregnate additives in ophthalmic articles consists of immersing the finished or semi-finished transparent ophthalmic article in a solution or dispersion containing the additives to impregnate. An aqueous solution or dispersion will usually be used. Organic solutions or dispersions can also be employed, mainly depending on additive and/or polymer or copolymer solubility or on other considerations such as, for example, solvent toxicity and its relative facility to be removed/evaporated. However, in order to cause mass transfer between the solution or dispersion and the ophthalmic article, high temperatures (up to 90 °C) are frequently used and, once again, this can degrade the additives, polymers and copolymers. Other important disadvantages of this method are the significant loss of additives and solvents and the difficulty associated with the need to completely removal the solvent used. Incomplete removal of the solvents is of extreme importance when the solvents used are dangerous or toxic. Finally, the impregnation of additives using this technique is usually a long and slow procedure which usually only produces superficial impregnation, i.e. the additives cannot penetrate very deeply inside the ophthalmic article.

Finally, a method of thermal transfer can also be used to impregnate additives into ophthalmic articles. This method consists of coating, by various means, the surface of the ophthalmic article with the additives to impregnate and subsequently thermally process the coated article in order to cause the diffusion of the additives and their consequent impregnation into the ophthalmic article. Like other thermal methods, this technique can also degrade heat sensitive additives, polymers or copolymers because it requires long heating and processing periods. Another disadvantage of this technique is the fact that it can cause modifications in the dimensions, geometry and physical and mechanical properties of the ophthalmic articles to be processed. Finally, an extremely important issue, this method does not permit a very deep impregnation into the ophthalmic article.

Additives can also be impregnated into polymers and copolymers by dissolving them in compressed high volatile solvent fluids, or mixtures thereof (such as carbon dioxide, ethylene, etc.), at temperatures and pressures near or above their critical temperatures and pressures, and subsequently contacting these g mixtures containing the dissolved additives with the polymers/copolymers to be impregnated. Under these conditions, the compressed fluid or fluid mixture can also act as a plasticizer and swelling agent for the polymers and copolymers, thus helping the diffusion of the additives in the polymers and copolymers.

U.S. Patents 4,598,006 and 4,678,684 describe a method to impregnate thermoplastic polymers with a given impregnation material (fragrance, pest control agent and pharmaceutical composition). This is done by dissolving the impregnation material in a volatile swelling agent, kept at or near supercritical conditions, contacting this mixture with the thermoplastic polymer, impregnating it and finally reducing pressure in order to precipitate the impregnation additive inside the thermoplastic polymer and release the volatile swelling agent. No reference is made to the impregnation of ophthalmic articles, contact lenses or hydrogels.

U.S. Patent 4,820,752 describes a method for infusing an additive into a polymer by dissolving the additive in a gaseous compressed fluid solvent. The additive should have some degree of solubility in the compressed fluid and in the polymer. The compressed fluid should have a regular boiling point below room temperature and a density of at least 0.01 gcm⁻³, and it should also be able to swell the polymer material. The solution containing the additive and the compressed fluid is then contacted with the polymer material, for a certain period of time, to enable it to absorb part of the solution. Finally, pressure is reduced, causing the release of the compressed fluid and the infusion of part of the additive in the polymer material. The document refers to the use of a supercritical fluid, but this condition it is not essential for the explained method. No reference is made to the impregnation of ophthalmic articles, contact lenses or hydrogels.

The U.S. documents 5,340,614 and 5,508,060 illustrate the impregnation of additives into a polymer substrate when the additives are insoluble in a supercritical fluid. The additives are dissolved in a liquid solvent, such as water, or in a liquid solvent containing a surfactant, and contacted with a polymer substrate in the presence of a supercritical fluid. Preferably, the liquid solvent should be considerably insoluble in the supercritical fluid. Releasing the pressure, the liquid solvent diffuses out of the swollen polymer substrate, leaving a certain amount of the additive trapped within the substrate. However, it is not clear that the aforesaid method will guarantee the complete removal of the liquid solvents and surfactants used, and no reference is made to the impregnation of ophthalmic articles, contact lenses and hydrogels.

Document WO 99/25322 describes a method for preparing pharmaceutical compositions in the form of powders of crosslinked polymers loaded with drugs and prepared using supercritical fluids. The process is very similar to those described in U.S. Patents 4,598,006, 4,678,684 and 4,820,752 but applied to crosslinked polymer materials in the form of powders, formulated as tablets, chips, pearls or granules for pharmaceutical use. No reference is made to ophthalmic articles, contact lenses or hydrogels.

Documents WO 95/20476 and WO 96/26059 describe methods for treating contact lenses made from polymerizable materials using supercritical fluids. These treatment processes comprise a method for dry releasing a contact lens from its holding implement (deblocking) and a method for extracting residual materials of non-polymerized monomers, organic solvents and some oligomers. No reference is made to the preparation of therapeutic ophthalmic articles.

Documents FR 2,795,082, WO 98/07054 and US 2002/0006469 A1 describe a method for incorporating photochromic additives into finished or semi-finished ophthalmic articles made of transparent organic polymers by means of the use of a fluid in the supercritical state. The supercritical fluid is introduced in a reactor containing the ophthalmic article and the photochromic additive to be impregnated, for a predetermined period of time, in order to allow impregnation to occur. After this time, the supercritical fluid is removed and the ophthalmic article is recovered with the photochromic additive impregnated at a certain depth. In the above-mentioned documents, no reference is made to the impregnation of drugs or drug compositions in order to prepare therapeutic ophthalmic articles.

### BRIEF DESCRIPTION OF THE DRAWINGS

This invention can additionally be illustrated by reference to the accompanying drawings wherein:
**Figure 1** describes a first diagram illustrating a laboratory installation that can be employed in the execution of this invention.
**Figure 2** describes a second diagram illustrating a laboratory installation that can be employed in the execution of this invention.
**Figure 3** describes a third diagram illustrating a laboratory installation that can be employed in the execution of this invention.
**Figure 4** is a graphic representation of the cumulative amount of Flurbiprofen released (mg) as a function of release time (hours), for the experiment described in Example 1.
**Figure 5** is a graphic representation of the cumulative amount of Flurbiprofen released (mg) as a function of release time (hours), for the experiment described in Example 2.
**Figure 6** is a graphic representation of the cumulative amount of Flurbiprofen released (mg) as a function of release time (hours), for the two experiments described in Example 3.

### SUMMARY OF THE INVENTION

This invention describes a method for preparing therapeutic ophthalmic articles using compressed fluids. Particularly, this invention refers to a method for impregnating a drug or a drug composition into finished or semi-finished transparent ophthalmic articles with a polymer base, such as contact lenses or intraocular lenses, or other biodegradable and/or biocompatible polymer matrices or hydrogels, through the use of compressed fluids, aiming at the preparation of drug sustained release systems for the treatment of glaucoma and other eye diseases. Other human pathologies can also be treated with these drug sustained release systems.

### DETAILED DESCRIPTION OF THE INVENTION

It is rather advantageous to have a method for the impregnation of drugs or drug compositions into finished or semi-finished ophthalmic articles, which does not alter and/or damage their physical, mechanical and optical properties (dimensions, shape, power, oxygen permeability, transmittance, water content and wettability) or degrades their constituent additives, polymers and copolymers. This makes it possible to have for example non-therapeutic finished or semi-finished ophthalmic articles, which will subsequently be impregnated them with the desired drugs or drug compositions according to the specific needs of patients. These impregnated ophthalmic articles can also be stored for future use.

A method with these characteristics can replace the traditional methods of drug impregnation of ophthalmic articles, such as those which use the polymerization reactions or impregnation by soaking in liquid non-volatile solvents, avoiding the typical disadvantages associated to each of them. Furthermore, it is desirable to have a method in which the drug or drug composition loading and impregnation depth can easily be controlled and where, at the end, the drug or drug composition is homogeneously dispersed in the polymer matrix of the ophthalmic article. Other important issues to be taken into consideration are: the drug or drug composition impregnation should be accomplished in relatively short treatment times and no dangerous solvent residues should remain in the impregnated ophthalmic articles.

The resulting ophthalmic articles can be used as drug sustained release systems for the treatment of several eye diseases and other human pathologies. These ophthalmic articles can be contact lenses or intraocular lenses, as well as other biocompatible and/or biodegradable solid polymer matrices or hydrogels. The drugs (and their salts) and drug compositions which can be impregnated in accordance with the present invention are those required for the final therapeutic purposes and must be soluble, to a certain extent, in the compressed fluid or compressed fluid mixture, or in the mixture of compressed fluid and a co-solvent to be used as swelling agent and plasticizer for the ophthalmic articles.

The present invention describes a method whereby therapeutic ophthalmic articles are prepared through the impregnation of drugs or drug compositions by means of compressed fluids.

More specifically, the present invention relates to a method for impregnating a drug or a drug composition into ophthalmic articles, aiming at the preparation of drug sustained release systems for the treatment of glaucoma, other eye diseases and other human pathologies wherein the therapeutic ophthalmic articles are prepared through the impregnation of drugs with compressed fluids or compressed fluid mixtures, or mixtures of compressed fluids and a co-solvent:
- the dissolution of a drug or drug composition in a compressed fluid or mixture of compressed fluids, or mixture of compressed fluids and a co-solvent, in liquid, sub-critical liquid, gaseous or supercritical state, in appropriate composition, temperature and pressure ranges;
- the contact of the resulting compressed fluid mixture and the ophthalmic articles, during an appropriate contact time, in appropriate composition, pressure and temperature ranges, and the subsequent impregnation of the drug or drug composition into the ophthalmic articles;
- the reduction of the pressure, after the contact time considered to be appropriate, and the subsequent removal of the compressed fluid or mixture of compressed fluids, or mixture of compressed fluids and a co-solvent, and the recovery of the ophthalmic articles impregnated with the drug or drug composition.

A first embodiment of the invention involves the following steps:
- introducing a compressed fluid or mixture of compressed fluids, or mixture of compressed fluids and a co-solvent, in a liquid, sub-critical liquid, gaseous or supercritical state, into a sealed high pressure vessel containing the ophthalmic article (or articles) and the drug or drug composition for impregnation. Inert and preferably non-toxic co-solvents can be added to increase the solubility of the drugs in the fluid mixture;
- retaining the compressed fluid or mixture of compressed fluids, or the mixture of compressed fluids and a co-solvent, inside the high pressure vessel under pre-established temperature and pressure operating conditions, with or without magnetic stirring, for a pre-determined period of time in order to cause the solubilization of the drug or drug composition in the compressed fluid or mixture of compressed fluids, or in the mixture of compressed fluids and a co-solvent, and impregnating the drug or drug composition in the ophthalmic article (or articles), at a pre-established depth and concentration;
- removing the compressed fluid or mixture of compressed fluids, or the mixture of compressed fluid and a co-solvent, through slow expansion from the high pressure vessel to atmospheric pressure, in order not to alter and/or damage the ophthalmic article (or articles);
- recovering the ophthalmic article, or articles, in which the drug or drug composition was impregnated at a certain depth and concentration.

A second embodiment of the invention involves the following steps:
- introducing a compressed fluid or mixture of compressed fluids, or mixture of compressed fluids and a co-solvent, in a liquid, sub-critical liquid, gaseous or supercritical state, into a first sealed high pressure vessel containing the drug or drug composition to be impregnated and the ophthalmic article (or articles) into a second sealed high pressure vessel;
- keeping the compressed fluid, or mixture of compressed fluids, or mixture of compressed fluid and a co-solvent, inside the first sealed vessel under pre-established operating temperature and pressure conditions, with or without magnetic stirring, for a pre-determined period of time in order to cause the solubilization of the drug or drug composition in the compressed fluid or mixture of compressed fluids, or in the mixture of compressed fluids and a co-solvent;
- transferring the resulting mixture from the first sealed vessel to the second sealed vessel, which contains the ophthalmic article (or articles) to be impregnated;
- retaining the mixture which contains the compressed fluid or mixture of compressed fluids, or the mixture of compressed fluids and a co-solvent, the drug or drug composition and the ophthalmic article or articles inside the second high pressure vessel under pre-established temperature and pressure operating conditions, with or without magnetic stirring, for a pre-determined period of time in order to cause the impregnation of the drug or drug composition in the ophthalmic article (or articles), at a pre-established depth and concentration;
- removing the compressed fluid or mixture of compressed fluids, or the mixture of compressed fluids and a co-solvent, through slow expansion from the second high pressure vessel to atmospheric pressure, in order not to alter and/or damage the ophthalmic article (or articles);
- recovering the ophthalmic article, or articles, in which the drug or drug composition was impregnated at a certain depth and concentration.

A third embodiment of the invention involves the following steps:
- introducing a compressed fluid or mixture of compressed fluids, or mixture of compressed fluids and a co-solvent, in a liquid, sub-critical liquid, gaseous or supercritical state, into a first sealed high pressure extractor containing the drug or drug composition, as a filler, and the ophthalmic article (or articles) into a second sealed high pressure vessel
- pumping and pressurizing the compressed fluid or compressed fluids mixture, or mixture of compressed fluids and a co-solvent, into the two sealed high pressure vessels, placed in contact at a certain pre-established pressure;
- closing the connection between the two sealed high pressure vessels;
- dissolving the drug or drug composition in the compressed fluid or mixture of compressed fluids, or in the mixture of compressed fluids and a co-solvent, in the first sealed high pressure extractor, under certain pre-established temperature and pressure operating conditions, without stirring, for a pre-established period of time;
- transferring the resulting mixture from the first sealed extractor to the second sealed vessel, which contains the ophthalmic article (or articles) to be impregnated;
- pressurizing the whole system until attaining a new predefined pressure and slowly opening the system expansion valve, in order to obtain a low flow rate;
- maintaining this constant low flow rate of the compressed fluid or mixture of compressed fluids, or mixture of compressed fluids and a co-solvent, through the entire system, under pre-established temperature and pressure operating conditions, with or without magnetic stirring in the second high pressure vessel, for a pre-determined period of time in order to cause the impregnation of the drug or drug composition in the ophthalmic article (or articles), at a pre-established depth and concentration;
- completely removing the compressed fluid or mixture of compressed fluids, or the mixture of compressed fluids and a co-solvent after the above-mentioned pre-established period of time, by means of a slow expansion of the system to atmospheric pressure, in order not to alter and/or damage the ophthalmic article (or articles);
- recovering the ophthalmic article or articles, in which the drug or drug composition was impregnated at a certain depth and concentration.

We can state that pure substances are generally known to exist in three physical states of matter: solid, liquid and gaseous. Pure substances can change from one to another of these three states by means of phase changes induced by alterations to their temperature and/or pressure. However, there is a point, at a certain temperature and pressure, beyond which substances can change from a liquid to a gaseous state without evaporation, or from a gaseous to a liquid state without condensation. This point is known as the critical point of a pure substance and it has temperature and pressure coordinates (*T*_{*c*}*, p*_{*c*}), being *T*_{*c*} the critical temperature and *p*_{*c*} the critical pressure.

A fluid in a state characterized by temperatures and pressures higher than its critical temperature and critical pressure respectively, in the case of a pure substance, or by temperatures and pressures above the boundary of critical points represented on a temperature-pressure-composition phase balance diagram, in the case of a mixture, is said to be in the supercritical state.

Herein we consider that a compressed fluid is a pure fluid at relatively high pressures that can exist in a liquid, sub-critical liquid, gaseous or supercritical state. We also consider that a mixture of compressed fluids is a mixture of two or more fluids at relatively high pressures ,which can exist in a liquid, sub-critical liquid, gaseous or supercritical state.

**Table 1** presents several non-limiting examples of fluids which can be useful in the method presented in this invention, as well as their critical temperatures and pressures. Other fluids can also be useful in the method described in this invention , such as: nitrogen, nitrous oxide, ethanol, methanol, sulfur hexafluoride, cyclohexane, methane, toluene, p-xylene, tetrafluoromethane, perfluoroethane, tetrafluoroethylene, 1,1-difluoroethylene. Additionally, mixtures of two or more fluids indicated above and in **Table 1** can be used.

| **Table 1 - Critical points for several fluids** | | |
|---|---|---|
| **Fluid** | ***T***_{***c***} **/ K** | ***p***_{***c***} **/ bar** |
| carbon dioxide | 304.1 | 73.8 |
| ethane | 305.4 | 48.8 |
| ethylene | 282.4 | 50.4 |
| propane | 369.8 | 42.5 |
| propylene | 364.9 | 46.0 |
| trifluoromethane | 299.3 | 48.6 |
| chlorotrifluoromethane | 302.0 | 38.7 |
| trichlorofluoromethane | 471.2 | 44.1 |
| n-pentane | 469.7 | 33.7 |
| water | 647.3 | 221.2 |
| ammonia | 405.5 | 113.5 |

In products for medical and pharmaceutical application, the presence of dangerous and/or toxic residual organic solvents is rigorously set and controlled by international organizations and safety regulations. Thus, it is necessary to guarantee the complete removal and absence of these substances, without exposing the present substances (polymers, copolymers and drugs) to high temperatures, which can thermally degrade them. Therefore, some compressed fluids are undoubtedly very attractive and useful alternative solvents.

Furthermore, compressed fluids, particularly supercritical fluids, have some liquid-like properties (density) and some gas-like properties (transport properties). As such, generally speaking this means that a supercritical fluid can easily penetrate inside solid matrices (including polymer matrices) and extract or precipitate substances in those matrices. This can be done simply by manipulating pressure. Additionally, supercritical fluids frequently have excellent plasticizing properties and can swell many polymer matrices, thus causing diffusion and consequently the extraction and impregnation processes to be carried out. Finally, another important advantage is the one resulting from the elimination of used compressed gaseous solvents and the recovery of the final products in an easy and cheap manner, when compared with traditional impregnation processes, leaving no trace of solvent residues.

Any of the above-mentioned compressed fluids and those indicated in **Table 1** or mixtures of these fluids are useful and can be applied to the method of the present invention. However, it is preferable to choose a compressed fluid or mixture of compressed fluids which is non-reactive and inert in respect of drugs or drug compositions and polymers and copolymers used in the method of this invention.

Preferably, the compressed fluid or mixture of these fluids should be very volatile or in a gaseous state under ambient conditions, in order to facilitate its removal by expansion and/or evaporation after the impregnation process takes place. This feature is also relevant to obtain short treatment and processing times. For safety reasons, the compressed fluid or mixture of these fluids, should preferably be non-toxic and non-flammable, as well as recyclable for reuse in the future.

Another important requirement for the method described in the present invention is that the compressed fluid or mixture of compressed fluids should be able to dissolve the drug or drug composition to a certain extent, so that it will be possible to carry out the impregnation of the drug or drug composition later on in the process. We can define the solubility of the drug or drug composition as the amount of solute (s) (drug or drug composition) that can be dissolved in a certain amount of solvent(s) (compressed fluid or mixture of compressed fluids) under certain temperature and pressure operating conditions. This solubility depends mainly on the chemical and physical similarity between the solute(s) and the solvent(s), pressure, temperature and other specific interactions that might exist between the substances present. Solubility also depends on the possible presence of impurities in the solute(s) and solvent(s). This fact makes it possible to increase the solubility of certain substances by adding small amounts of co-solvents to the mixture, or to decrease it, adding small amounts of anti-solvents to the mixture. Usually, for the method of this invention, in order to increase the solubility of a drug or drug composition in a compressed fluid or mixture of compressed fluids, one should choose a highly volatile co-solvent which has low or no toxicity and is capable of increasing this solubility. Typical non-limiting examples of useful co-solvents for the method of this invention are low molecular weight alcohols, such as methanol or ethanol, which is less used.

Frequently, compressed fluids and in particular supercritical fluids are known as "tunable" solvents because, given that it is possible, merely by manipulating pressure, to change the density of the medium and consequently its capability to dissolve or precipitate substances. Increasing pressure usually increases the solubility of the drug or drug composition. However, the effect of temperature on the solubility of the drug or drug composition is not so obvious in view of the effect known as temperature crossover. Within certain pressure ranges, an increase in temperature will increase solubility, but within other pressure ranges it will decrease solubility. Therefore, for the method of the present invention and its application, development and optimization, it is necessary to have an experimental, precise and detailed knowledge of the solubility of the drug or drug composition in the compressed fluid or mixture of compressed fluids.

On the other hand, the compressed fluid, mixture of compressed fluids or mixture of compressed fluid and a co-solvent should not be able to dissolve the finished or semi-finished ophthalmic articles, polymer matrices and hydrogels used in the method described in this invention. However, they should have some solubility and be capable of penetrating and swelling, to a certain extent, these solid articles and matrices. This will decrease their glass transition temperature (*T*_{*g*}), thus helping and causing the diffusion of the additive (drug or drug composition) into their constituent polymers and copolymers. This makes it possible to increase the loading of the additives, as well as their penetration depth and homogeneous dispersion through the polymer substrates.

Finally, it is also of extreme relevance that the compressed fluid or mixture of compressed fluids do not alter and/or damage the physical, mechanical and optical properties (such as dimensions, shape, power, oxygen permeability, transmittance, water content and wettability) of the finished or semi-finished ophthalmic articles, polymer matrices and hydrogels used in the method of this invention.

Therefore, the temperature and pressure operating conditions for the method of the present invention can be varied and should be chosen based on the following main criteria: critical point of the compressed fluid (or mixture of compressed fluids), solubility of the drug (or drug composition) in the compressed fluid (or mixture of compressed fluids) and solubility of the compressed fluid (or mixture of compressed fluids) in the ophthalmic articles. However, it is advisable that the operating temperature of the compressed fluid or mixture of compressed fluids is 30 °C or more, preferably 50 °C, lower than the glass transition temperature under normal conditions of the polymers or copolymers which compose the ophthalmic articles to be processed. Other important issues to be taken into consideration in order to avoid both the thermal degradation and the alteration of physical, mechanical and optical properties (dimensions, shape, power, oxygen permeability, transmittance, water content and wettability) of polymers, copolymers and drugs, due to too fast heating/cooling and compression/decompression cycles.

For various reasons, carbon dioxide (CO₂) is an advantageous and useful fluid to be used in the method of the present invention. It has a critical point relatively easy to attain (*T*_{*c*} = 304.1 K and *p*_{*c*} = 73.8 bar), which can avoid the degradation of thermally labile substances, it is non-toxic and non-flammable, has a non-polluting character, relatively low cost and it is abundant in nature. Furthermore, it is relatively inert with most chemical species and shows excellent plasticizing and swelling properties in respect of several polymers and copolymers.

Therefore, whenever possible and advantageous, the use of carbon dioxide is recommended for the method described in this invention. However, among other fluids that can be used in accordance with the invention, a special reference should be made to nitrous oxide, sulfur hexafluoride, ethylene, ethane, propane and chlorotrifluoromethane.

The ophthalmic articles which can be treated by the method described in this invention can be finished or semi-finished articles made of a transparent polymer substrate or articles made of other biocompatible and/or biodegradable solid polymer matrices or hydrogels. They can consist of intra-ocular lenses, hard (RGP) or soft contact lenses, hydrophobic, hydrogel contact lenses, hydrophilic, in their dry or wet state, and other solid polymer matrices or hydrogels of interest to the method presented in this invention.

Finished ophthalmic articles are polymer-based transparent ophthalmic articles in their final shape and dimension, usually prepared by polymerization reactions between molds and with their two faces at the finishing stage. Semi-finished ophthalmic articles are polymer-based transparent ophthalmic articles which, after being prepared and molded, show a single face at the finishing stage, being possible to treat the surface of the other face with a subsequent finishing. Other biocompatible and/or biodegradable transparent or non-transparent polymer matrices or hydrogels are other linear, branched or crosslinked polymers, copolymers or mixtures thereof, suitable for the method described in this invention.

The ophthalmic articles which can be used in the method described in this invention are made of linear, branched or crosslinked polymers, copolymers or mixtures thereof, appropriate for manufacturing an ophthalmic article. Several non-limiting examples of these polymers, copolymers or mixtures thereof are: poly(acrylates), poly(hydroxyalkyl acrylates), poly(methacrylates), poly(hydroxyalkyl methacrylates), poly(alkyl acrylates), poly(alkyl methacrylates), poly(allyl methacrylates), poly(diacrylates), poly(triacrylates), poly(dimethacrylates), poly(trimethacrylates), poly(alkyl diacrylates), poly(glycerol methacrylates), poly(aminoalkyl acrylates), poly(aminoalkyl methacrylates), poly(aminoalkyl diacrylates), poly(aminoalkyl dimethacrylates), poly(ethoxybiphenol-A dimethacrylates), poly(ethyleneglycol methacrylates), poly(ethyleneglycol dimethacrylates), poly(ethyleneglycol trimethacrylates), poly(siloxanyl acrylates), poly(siloxanyl methacrylates), poly(siloxanyl alkyl acrylates), poly(siloxanyl alkyl methacrylates), poly(siloxanyl alkyl diacrylates), poly(siloxanyl alkyl dimethacrylates), poly(sylil methylene methacrilates), poly(siloxanyl alkyl vinyls), poly(siloxanyl alkyl carbamate-carbonates), poly(siloxanyl alkyl itaconates), poly(siloxanyl alkyl alkynes), poly(siloxanyl alkyl fumarates), poly(fluoro alkyl acrylates), poly(fluoro alkyl methacrylates), poly(fluoro alkyl dimethacrylates), poly(fluoro alkyl diacrylates), poly(fluoro alkyl trimethacrylates), poly(fluoro alkyl itaconates), poly(fluoro alkyl mesoconates), poly(fluoro ethers), poly(urethanes), poly(thiourethanes), poly(carbonates), poly(allyl carbonates), poly(N-vinyl pyrrolidinones), poly(diacetone acrylamides), poly(alkyl acrylamides), poly(dialkyl acrylamides), poly(hydroxyalkyl acrylamides), poly(methacrylamides), poly(acrylic acids), poly(methacrylic acids), poly(ethers), poly(esters), poly(vinyl esters), poly(orthoesters), poly(vinyl alcohols), poly(ethylene terephthalates), poly(butylene terephthalates), hydrolyzed poly(acrylonitriles), poly(siloxanes), poly(vinyl acetals), poly(phospazenes), poly(ethylene glycols), poly(propylene glycols), poly(butylene glycols), poly(ethylene oxides), poly(propylene oxides), poly(butylene oxides), poly(vinyl acetates), poly(vinyl butyrates), poly(hyaluronic acids), poly(imines), poly(ethylene imines), poly(imines), poly(amines), poly(vinyl amines), poly(caprolactones), poly(lactic acids), poly(lactic-co-glycolic acids), poly(hydroxyl alkanoates), poly(styrenes), poly(t-butyl styrenes), poly(vinyl benzenes), poly(divinylbenzenes), poly(fluorostyrenes), poly(sulfonamides), poly(sulfonates), poly (allyl sulfonates), poly(anhydrides), cellulose acetate, cellulose triacetate, cellulose acetate propionate, cellulose acetate butyrate, chitin, chitosan, modified chitosan, dextran, modified dextran, starch, modified starch, etc. Other polymers, copolymers and mixtures thereof which can also be used as ophthalmic articles are widely described in references texts, such as documents WO 95/10790 and EP-A1-0653428.

Among the drugs (and salts thereof) which can be impregnated according to the invention and used for the treatment of eye diseases, we can present the following non-limiting examples: steroid and corticosteroid anti-inflammatories (prednisolone, methylprednisolone, fluorometholone, dexamethasone, betamethasone, hydrocortisone, medrysone, loteprednol, rimexolone, triamcinolone), non-steroid anti-inflammatories and anti-allergenics (diclofenac, ketorolac, flurbiprofen, indomethacin, suprofen, ibuprofen, ketorolac tromethamine, emedastine, levocabastine, azelastine, olopatadine, ketotifen, cromolyn, lodoxamide), beta-adrenergic receptor antagonists (timolol maleate, timolol hemihydrate, metipranolol, carteolol, betaxolol, levabunolol, levobetaxolol), alpha-adrenergic receptor antagonists (brimonidine, apraclonidine), prostaglandin analogues (latanoprost, travoprost, bimatoprost), docosanoids (unoprostone isopropyl), cholinergic agonists (pilocarpine, carbachol), adenergic antagonists (epinephrine, dipivefrin, epinephryl borate), carbonic anhydrase inhibitors (dorzolamide, acetazolamide, dichlorphenamide, methazolamide, brinzolamide), vitamins and mineral supplements (vitamin A (beta-carotene), vitamin C (ascorbic acid), vitamin E (dl-alpha-tocopheryl acetate), zinc (zinc oxide), copper (cupric oxide), antibiotics (penicillins and synthetic penicillins (amoxicillin, dicloxacillin, augmentin), cephalosporins (1^{st}, 2^{nd}, and 3^{rd} generations), aminoglycosides (gentamycin, tobramycin, neomycin), macrolides (erythromycin, azithromycin, clarithromycin), fluoroquinolones (ofloxacin, ciprofloxacin, norfloxacin, levofloxacin)), antivirals (acyclovir, valacyclovir, famcyclovir), analgesics (diclofenac, acetaminophen, acetylsalicylic acid, ibuprofen, celecoxib, rofecoxib, valdecoxib, tramadol), antihistamines (diphenhydramine, chlorpheniramine, cetirizine, loratadine, desloratadine, fexofenadine), mydriatic and dilation reversal drugs (tropicamide, hydroxyamphetamine, dapiprazole), and mixtures of two or more of the above-mentioned drugs.

For the treatment of other human pathologies, we can state the following non-limiting examples of therapeutic drug types which can be impregnated in accordance with the method of the present invention: antifungals, antibacterials, antivirals, anti-inflammatories, antibiotics, corticosteroids, antirheumatic agents, relaxants, anesthetics, analgesics, antipyretics, antiepileptics, anti-Parkinson drugs, psycholeptics, anxiolytics, hypnotic and sedative drugs, antiparasitics, antihistamines, immunoglobulins, insulins and analogues thereof, electrolytes with carbohydrates, blood glucose lowering drugs, vitamins, mineral supplements, tonics and nutrients, anabolic agents, digestives, laxatives, appetite suppressants and stimulants, antithrombotic agents, antihemorrhagic drugs, antianemic preparations, cardiac glycosides, antiarrhythmics, cardiac stimulants, vasodilators, antihypertensives, diuretics, vasoprotectives, alpha- and beta-blocking agents, calcium channel blockers, UV-radiation protectors, antiseptics, contraceptives, sex hormones and other hormonal preparations, vaccines, peptidic molecules, antineoplastic agents, aminoacids, diagnostic agents and contrast agents, or any mixtures of two or more of the above-mentioned types of drugs.

The amount of drug (or drug composition) for impregnation into the ophthalmic articles to be processed can be directly controlled through their concentration in the compressed fluid, mixture of compressed fluids or mixture of compressed fluid with a co-solvent. Generally, the higher the concentration of the drug or drug composition in the compressed mixture, the higher will be the drug or drug composition concentration in the ophthalmic articles processed by the method of this invention. However, this concentration is indirectly controlled by the degree of solubility of the drug or drug composition in the compressed fluid or mixture of compressed fluids, and it cannot be too high, since it can negatively affect the swelling and plasticizing behavior of the compressed mixture.

Like in the case of the compressed fluid or mixture of compressed fluids the drug (or drug composition) to be impregnated into the ophthalmic articles must not alter and/or damage the physical, mechanical and optical properties (dimensions, shape, power, oxygen permeability, transmittance, water content and wettability) of the ophthalmic articles, polymers, copolymers and drugs used in this invention.

The length of treatment or time required for the method described in this invention will mainly depend on the amount and depth of impregnation into the ophthalmic articles to be processed desired for the drug (or drug composition). In general, longer periods of treatment will favor a deeper impregnation and a higher amount of drug (or drug composition) impregnation.

Evidently, the final concentration and impregnation depth results should always be a function of the final use desired for the ophthalmic article (i.e. daily, weekly, monthly bimonthly use, etc.) and drug levels intended for therapeutic action in patients. Furthermore, they will obviously also depend on the nature of the polymers and copolymers (chemical and physical nature, molecular weight, degree of cross-linking and branching, degree of crystallinity, density, orientation, etc.), compressed fluids (or mixture of compressed fluids), co-solvents and drugs (or drug compositions), the specific interactions that may occur between all the contacted substances, the operating conditions applied (temperature, pressure, cell volumes, concentrations, etc.) and other important characteristics of the ophthalmic articles, such as dimension and geometry (e.g. ratio between external surface area and volume).

As referred to above, a generic method for implementation of the present invention is described herein, as well as three embodiments thereof. It is obvious that the treatment time will also depend on the nature and characteristics of each one if these embodiments. The embodiments of the invention are typically discontinuous processes. The last embodiment can be considered as semi-continuous with regard to the compressed fluid (or mixture of compressed fluids). However, the second and third embodiments of the invention can be optimized in order to reduce processing times (e.g. pre-pressurizing the vessels containing the ophthalmic articles to be impregnated, which will result in pre-swelling and pre-plasticization thereof, thus facilitating the impregnation to be carried out afterwards).

Typical processing times to obtain the desired drug (or drug composition) impregnation amount and penetration depth in the ophthalmic article vary between 30 minutes and 3 hours, depending on the embodiment of the invention implemented and the above-mentioned factors. However, and it has also been mentioned, these times can be optimized in certain circumstances, usually leading to processing times within the game of 30 to 60 minutes.

Another very important factor for the method of the present invention is the final step of system decompression down to atmospheric pressure. Once the impregnation of the drug (or drug composition) into the ophthalmic articles is completed, the system has to be completely depressurized, leaving the impregnated articles behind. The compressed fluid, mixture of compressed fluids or mixture of a compressed fluid and a co-solvent can easily be removed from the system; however, this process must be carried out very slowly and carefully, in order not to alter and/or damage the chemical, physical, mechanical and optical properties (dimensions, shape, power, oxygen permeability, transmittance, water content and wettability) of the finished or semi-finished ophthalmic articles, polymers, copolymers and drugs used in the method of this invention.

In view of the high volatility of the compressed fluid, mixture of compressed fluids or mixture of a compressed fluid and a co-solvent, their separation from the ophthalmic articles is easily achieved merely by system pressure reduction. However, if this depressurization is done too quickly, some microvoids, or even macrocavities, can be created, which can alter and/or damage some of the physical, mechanical and optical properties of the ophthalmic articles. Moreover, these cavities tend to form in the interior of the processed ophthalmic articles, not on their surfaces, thus preventing any possible final surface treatment for removing them. These voids can also act as undesirable "reservoirs" for the impregnation drug or drug composition, thus provoking heterogeneous impregnation and possible alteration of the necessary transparency of the ophthalmic articles.

The extent of these possible alterations will mainly depend on the depressurization rate but also on the operating pressure and temperature conditions, the amount of impregnated drug (or drug composition), the chemical and physical nature of all contacted materials, and the dimension and geometry of the ophthalmic articles. Therefore, all these parameters are of paramount importance and should be taken into consideration in the present invention.

The method described in this invention shows many advantages with regard to traditional methods for the preparation of therapeutic ophthalmic articles, which can be employed as drug sustained release systems for the treatment of several eyes diseases and other human pathologies.

As referred to above, the method will make it possible to obtain finished or semi-finished non-therapeutic ophthalmic articles and their subsequent impregnation with the desired drugs or drug compositions, according to the needs of patients. This particular feature can make it possible to develop very useful medical applications, which can also be commercially very attractive. Furthermore, the therapeutic ophthalmic articles prepared using this method can be stored in an appropriate medium for future therapeutic use.

Given that some of the specific operating characteristics of the method of the present invention, this impregnation method will not thermally alter and/or damage the physical, mechanical and optical properties (dimensions, shape, power, oxygen permeability, transmittance, water content and wettability) of the ophthalmic articles, nor will it degrade their constituent additives, polymers and copolymers.

Furthermore, in this method it is easy to control the loading and impregnation depth of the drug or drug composition, and at the end the drug or drug composition will be homogeneously dispersed in the polymer matrix of the ophthalmic article. Another very important feature is the fact that the impregnation of the drug or drug composition can be carried out within relatively short treatment times, leaving no dangerous solvent residues in the impregnated ophthalmic articles. The recovery of the final products is an easy and simple procedure, merely carried out by slowly expanding the system, and the products are recovered at a dry solvent-free final stage, no additional drying/evaporating step being required (and without the energy and operating time costs associated with these processes).

Finally, the method of the present invention makes it possible to reduce several operating costs. As such, it is possible to recover and reuse, in a simple and easy manner, the compressed fluids employed and the remaining amount of drugs which were not impregnated. For relatively expensive products, such as pharmaceutical products, this is an important financial advantage.

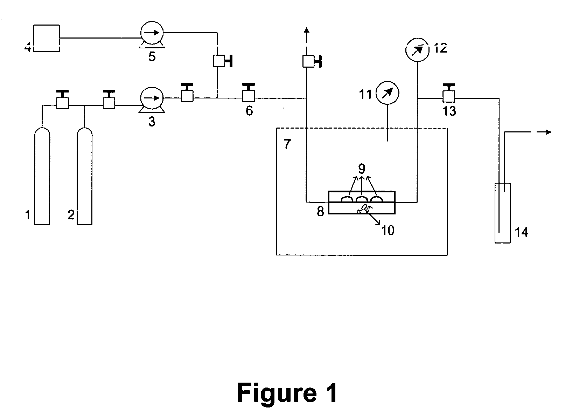

The method described in this invention is additionally illustrated and described by the following non-limiting examples. The impregnation of a drug or a drug composition into ophthalmic articles was conducted using laboratory equipment, as represented in the diagrams of **Figure 1, Figure 2** and **Figure 3**.

In accordance with **Figure 1**, a first embodiment of the invention consists of:
- introducing a compressed fluid **1** or mixture of compressed fluids **2** by means of a compressor/liquid pump **3** in an sealed high pressure vessel **8**, placed in a temperature controlled bath or oven **7**, containing the ophthalmic article (or articles) **9**, and the drug or drug composition to be impregnated. An inert and preferably non-toxic co-solvent **4** can be added by means of a compressor/liquid pump 5. Valve **6** is kept open and valve **1**3 is kept closed. The ophthalmic article **9** is placed on the top side of a perforated shelf and the drug or drug composition is placed at the bottom of the vessel;
- closing valve **6** and retaining the compressed fluid, or mixture of compressed fluids, or mixture of compressed fluid and co-solvent, in a liquid, sub-critical liquid, gaseous or supercritical fluid state inside vessel **8**, under pre-established temperature **11** and pressure **12** operating conditions, in a static or stirred mode **10**, for a pre-determined period of time, in order to cause solubilization and the impregnation of the drug or drug composition into the ophthalmic article (or articles) **9**, at predetermined depth and concentration;
- after this pre-determined period of time, opening valve **13** and completely removing the compressed fluid or mixture of compressed fluids, or mixture of compressed fluid and co-solvent, by slowly expanding the pressure vessel in order not to alter/damage the ophthalmic article (or articles) **9**. The amount of drug or drug composition not impregnated during the process is recovered in a cooled (or not cooled) trap **14**;
- opening vessel **8** and recovering the ophthalmic article (or articles) **9**, in which the drug or drug composition was impregnated at a certain depth and concentration.

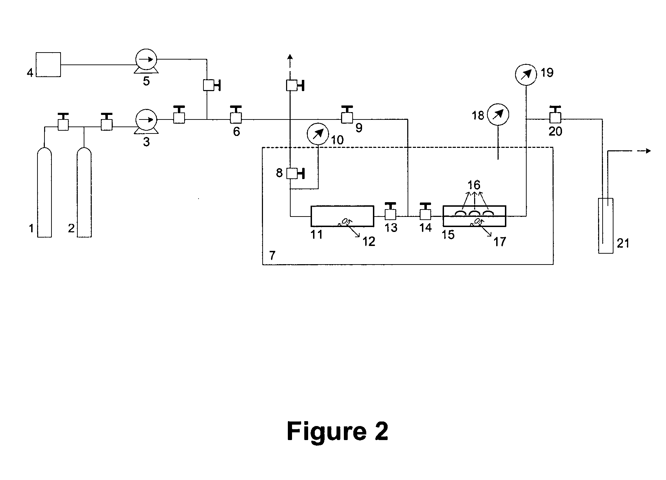

In accordance with **Figure 2**, a second embodiment of the invention consists of:
- introducing a compressed fluid **1** or mixture of compressed fluids **2** by means of a compressor/liquid pump **3** into a first sealed high pressure vessel **11**, placed in a temperature controlled bath or oven **7**, containing the drug or drug composition to be impregnated. An inert and preferably non-toxic co-solvent **4** can be added by means of a compressor/liquid pump **5**. Valves **6** and **8** are kept open and valves **9, 13, 14** and **20** are kept closed. The ophthalmic articles **16** are placed on the top side of a perforated shelf, in a second sealed high pressure vessel **15**, also in the same temperature controlled bath or oven **7**;
- closing valves **6** and **8** and retaining the compressed fluid or mixture of compressed fluids, or mixture of compressed fluid and co-solvent, in a liquid, sub-critical liquid, gas or supercritical fluid state, inside the first sealed vessel **11**, under pre-established temperature **18** and pressure **10** operating conditions, in a static or stirred mode **12,** for a pre-determined period of time in order to cause the solubilization of the drug or drug composition in the compressed fluid or mixture of compressed fluids, or mixture of compressed fluid and co-solvent;
- while the solubilization of the drug or drug mixture is taking place, a pre-swelling of the ophthalmic article (or articles) **16** can be carried out by opening valves **6** and **9** and introducing the compressed fluid or mixture of compressed fluids or mixture of compressed fluid and co-solvent, in the second high pressure vessel **15**, under certain pre-established temperature **18** and pressure **19** operating conditions. The pressure in the second sealed vessel **19** should be lower than the pressure in the first sealed vessel **10**;
- once the drug or drug composition is dissolved to a certain extent, transferring the resulting mixture from the first sealed high pressure vessel **11** to the second sealed high pressure vessel **15**, opening valves **13** and **14** and keeping valves **6, 8, 9** and **20** closed;
- after mixture transfer, closing valves **13** and **14** and retaining the resulting mixture containing the compressed fluid or mixture of compressed fluids, or mixture of compressed fluid and co-solvent, with the drug or drug composition inside the second sealed vessel **15**, under certain pre-established temperature **18** and pressure **19** operating conditions, in a static or stirred state **17**, for a pre-determined period of time in order to cause the impregnation of the drug or drug composition into the ophthalmic article (or articles) at a certain depth and concentration;
- after this pre-determined period of time, opening valve **20** and completely removing the compressed fluid or mixture of compressed fluids, or mixture of compressed fluid and co-solvent, by slowly expanding the pressure vessel in order not to alter/damage the ophthalmic article (or articles) **16**. The amount of drug or drug composition not impregnated during the process is recovered in a cooled (or not cooled) trap **21**;
- opening the sealed vessel **15** and recovering the ophthalmic article (or articles) **16**, in which the drug or drug composition was impregnated at a certain depth and concentration.

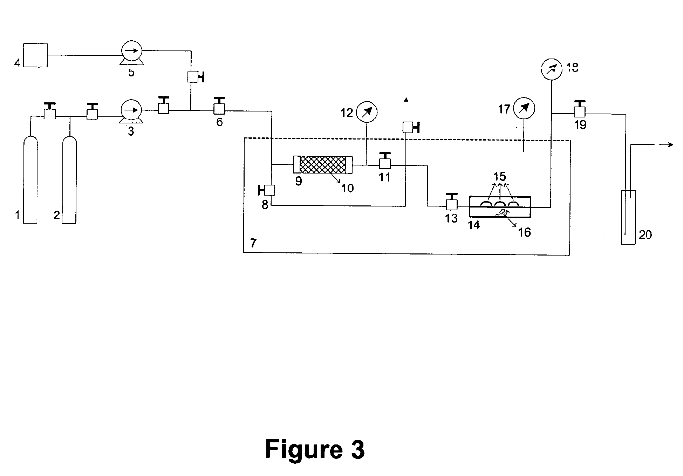

In accordance with **Figure 3**, a third embodiment of the invention consists of:
- introducing a compressed fluid 1 or mixture of compressed fluids **2** by means of a compressor/liquid pump **3** in a first sealed high pressure tubular extracting vessel **9**, placed in a temperature controlled bath or oven **7**, containing the drug or drug composition to be impregnated, packed between a sequence of filter paper, cotton, and perforated metal discs **10**. An inert and preferably non-toxic co-solvent **4** can be added by means of a compressor/liquid pump **5**. The ophthalmic articles **15** are placed on the top side of a perforated shelf, in a second sealed high pressure vessel **14**, also in the same temperature controlled bath or oven **7**. Valves **6, 11** and **13** are kept open, so that the two vessels **9** and **14** directly contact, and valves **8** and **19** are kept closed, under some pre-established temperature **17** and pressure **12** and **18** operating conditions;
- closing the connection between vessels **9** and **14** by closing valves **11** and **13**;
- closing valve **6** and retaining the compressed fluid or mixture of compressed fluids, or mixture of compressed fluid and co-solvent, in a liquid, sub-critical liquid, gaseous or supercritical fluid state inside the sealed tubular extractor **9**, under pre-established temperature **17** and pressure **12** operating conditions, in a static mode, for a pre-determined period of time in order to cause the solubilization of the drug or drug composition in the compressed fluid or mixture of compressed fluids, or mixture of compressed fluid and co-solvent, to a certain extent;
- transferring the resulting mixture from the sealed tubular extractor **9** to the second sealed high pressure vessel **14**, which contains the ophthalmic article (or articles) **15** to be impregnated, by opening valves **11** and **13**;
- opening valve **6** and pressurizing the whole system until attaining a new predefined pressure **12** and **17**, and slowly opening the expansion valve **19** at a very low flow rate;
- maintaining this constant low flow rate of compressed fluid or mixture of compressed fluids, or mixture of compressed fluid and co-solvent, through the entire system, under some pre-established temperature **17** and pressure **12** and **18** operating conditions, in static mode in tubular extractor **9**, and in static or stirred mode **16** in the second vessel **14**, for a pre-determined period of time in order to cause the impregnation of the drug or drug composition into the ophthalmic article (or articles) at a pre-established depth and concentration;
- after this pre-determined period of time, closing valve **6** and opening valve **19**, and completely removing the compressed fluid or mixture of compressed fluids, or mixture of compressed fluid and co-solvent, by slowly expanding the pressure vessel in order not to alter/damage the ophthalmic article (or articles) **15**. The amount of drug or drug composition not impregnated during the process is recovered in a cooled (or not cooled) trap **20**;
- opening the sealed vessel **14** and recovering the ophthalmic article (or articles) **15**, in which the drug or drug composition was impregnated at a certain depth and concentration.

The invention will be additionally illustrated by the three following non-limiting examples.

### Example 1

A Focus® Monthly® (Ciba Vision, GA, USA) hydrogel contact lens (ionic, high hydration, FDA Group IV, 55% water + 45% Vifilcon A copolymer) was treated by means of the above-mentioned first embodiment of the process, in accordance with the invention and according to **Figure 1**. The ophthalmic article was contacted in a sealed high pressure vessel with a mixture of compressed Carbon Dioxide (99.998% purity, Air Liquide), Flurbiprofen (CAS[5104-49-4], 97% purity, Sigma-Aldrich) and Ethanol (CAS[64-17-5], 99.8% purity, Fluka), as co-solvent (5% molar in respect of Carbon Dioxide). The operating conditions were 90.0 bar and 40.0 °C. No stirring was employed. After a 30 minute processing time, the vessel was slowly expanded (for 15 minutes) and the compressed fluid (Carbon Dioxide) was removed. Part of the co-solvent (Ethanol) was dragged out of the system by the Carbon Dioxide, while its remaining part precipitated, as a liquid, to the bottom of the high pressure vessel. The high pressure vessel was then removed from the temperature controlled bath, opened and the ophthalmic article was recovered, impregnated with Flurbiprofen.

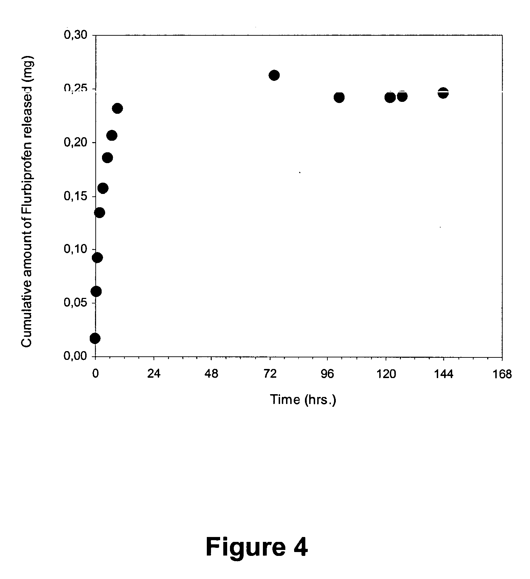

Later on, the impregnated ophthalmic article was placed in 6 ml of physiological serum (saline solution, 0.9% NaCl) inside a closed vessel. This vessel was subsequently placed inside a temperature controlled bath (at 37 °C), in order to carry out the in vitro sustained release kinetic study of the drug (Flurbiprofen). At regular time intervals (shorter time intervals at the beginning), 3 ml of the release solution (containing the released drug) were removed from the vessel and replaced by 3 ml of fresh physiological serum. The concentration of Flurbiprofen was determined by spectroscopy for each sample collected. The results are shown in **Figure 4**.

The maximum cumulative amount of released Flurbiprofen was about 0.25 mg. This amount was actually attained after 24 hours of release studies. For longer release times, this result remains almost constant, thus it can be assumed that this amount corresponds to the total amount of Flurbiprofen impregnated in the ophthalmic article.

### Example 2

A Focus® Monthly® (Ciba Vision, GA, USA) hydrogel contact lens (ionic, high hydration, FDA Group IV, 55% water + 45% Vifilcon A copolymer) was treated by means of the above-mentioned first embodiment of the process, in accordance with the invention and according to **Figure 1**. The ophthalmic article was contacted in a sealed high pressure vessel with a mixture of Carbon Dioxide (99.998% purity, Air Liquide) and Flurbiprofen (CAS[5104-49-4], 97% purity, Sigma-Aldrich). No co-solvent was added. The operating conditions were 90.0 bar and 40.0 °C. No stirring was employed. After a 30 minute processing time, the vessel was slowly expanded (for 15 minutes) and the compressed fluid (Carbon Dioxide) was removed. The high pressure vessel was then removed from the temperature controlled bath, opened and the ophthalmic article was recovered, impregnated with Flurbiprofen.

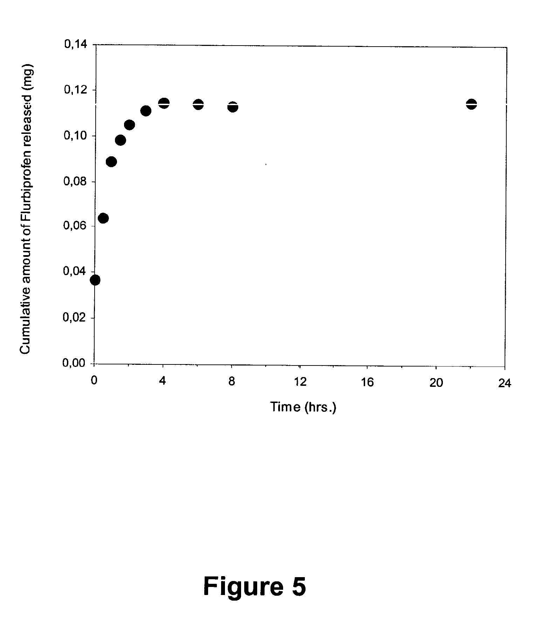

The in vitro kinetic studies concerning the release of Flurbiprofen were conducted in accordance with the experimental procedures already described at Example 1. The results of these studies are shown in **Figure 5**.
The maximum cumulative amount of released Flurbiprofen was about 0.11 mg. This amount was actually attained after 6 hours of release kinetic studies. For longer release times, this result remains almost constant, thus it can be assumed that this amount corresponds to the total amount of Flurbiprofen impregnated in the ophthalmic article. As it can be seen, this amount is almost half the amount obtained in Example 1, in which operating conditions (pressure, temperature and processing time) were identical but Ethanol was used as co-solvent.

### Example 3

Two Focus® Dailies® (Ciba Vision, GA, USA) hydrogel contact lenses (non ionic, high hydration, FDA Group II, 69% water + 31% Nelfilcon A copolymer) were separately treated in two different experiments (I and II) by means of the above-mentioned third embodiment of the process, in accordance with the invention and according to **Figure 3.** In both experiments, the ophthalmic articles processed were impregnated with Flurbiprofen (CAS[5104-49-4], 97% purity, Sigma-Aldrich) using Carbon Dioxide (99.998% purity, Air Liquide). No co-solvent was added and no stirring was employed.

In experiment I, the operating conditions were 41 °C and 90 bar, while in experiment II the operating conditions were 41 °C and 120 bar. In both experiments, drug solubilization was carried out for 1 hour and 30 minutes, and impregnation was carried out for 1 hour. After impregnation, the vessel was slowly depressurized (for 15 minutes) and the compressed fluid (Carbon Dioxide) was removed. The high pressure vessel was then removed from the temperature controlled bath, opened and the ophthalmic article was recovered, impregnated with Flurbiprofen.

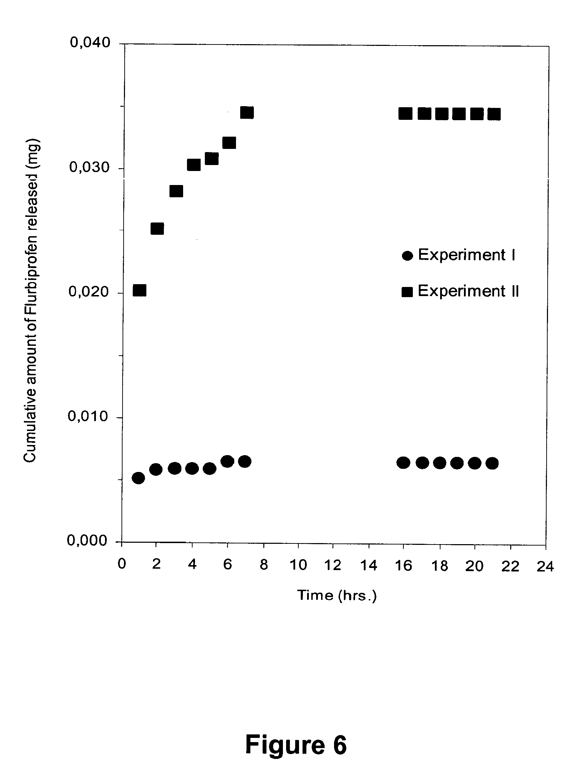

Afterwards, the two ophthalmic articles were stored and preserved in physiological serum (saline solution, 0.9% NaCl) in closed vessels, for three months. After this period, in vitro kinetic studies were carried out in respect of Flurbiprofen release, following the experimental procedures already described at Example 1. The results of these studies are shown in **Figure 6**.

The maximum cumulative amount of released Flurbiprofen was about 0.007 mg for experiment I and about 0.035 mg for experiment II. These amounts were actually attained after 7 hours of release kinetic studies. For longer release times, these results remain almost constant. However, in these cases it cannot be assumed that these values correspond to the total amount of Flurbiprofen impregnated in each ophthalmic article, given that part of the impregnated drug was released in the preservation medium during the three-month storage period. Preservation and storage media were also analyzed and the mass of Flurbiprofen released therein was about 0.0030 mg for experiment I and about 0.0025 mg for experiment II. Therefore, we can assume that the effective total mass of Flurbiprofen impregnated in the two articles was 0.0100 mg and 0.0375 mg respectively for experiments I and II.

It is therefore evident that, even after a three-month storage period, the ophthalmic articles are still impregnated with Flurbiprofen. During this period, part of the drug diffused out of the ophthalmic articles until chemical balance with the drug present in the storage solution was attained. This fact is of extreme importance for the development of pharmaceutical commercial applications based on this invention.

As observed and foreseen, the impregnation of Flurbiprofen is higher at higher operating pressure (experiment II) for the same operating temperature and length of treatment. This illustrates one of the important advantages of the invention: the possibility to easily control, by means of pressure manipulation, the amount of drug (or drug composition) to be impregnated into the ophthalmic articles. Other operating parameters can also be manipulated for impregnating the desired amount of the drug or drug composition: temperature, addition of co-solvents, solubilization and impregnation times. However, the amounts to impregnate and subsequently release must always take into consideration the therapeutic and toxic limits of any given drug or drug composition. For example, with regard to Flurbiprofen reference texts indicate that in baboons there is no acute toxicity (single dose of 125 mg/Kg) and that chronic toxicity is 5mg/Kg/day, for 26 weeks. On the other hand, the minimum amount of Flurbiprofen to be kept in the eye must be at least 0.03-0.15µg, in order to prevent inflammatory processes (M. Mariz, *MSc Thesis,* University of Coimbra, Portugal, 1999).

### References

### Patents

U.S. Patent 4,598,006 - Sand
U.S. Patent 4,678,684 - Sand
U.S. Patent 4,820,752 - Berens et al.
U.S. Patent 5,340,614 - Perman et al.
U.S. Patent 5,508,060, - Perman et al.
WO 99/25322 - Alessi et al.
WO 95/20476 - Bawa et al.
WO 96/26059 - Hoffman et al.
FR 2,795,082 - Baillet et al.
WO 98/07054 - Baillet et al.
U.S. Patent 2002/0006469 A1 - Baillet et al.
WO 95/10790 - Coleman et al.
EP 0653428 A1 - Guglielmetti et al.
M. Mariz, *MSc Thesis*, University of Coimbra, Portugal, 1999.

## Claims

1. A method for the impregnation of a drug or drug composition into ophthalmic articles in order to prepare drug sustained release systems for the treatment of glaucoma, other eye diseases and other human pathologies, **characterized in that** the therapeutic ophthalmic articles are prepared by the impregnation of drugs or drug compositions using compressed fluids or mixtures of compressed fluids, or mixtures of compressed fluids and a co-solvent, comprising the following steps:
- the dissolution of a drug or drug composition in a compressed fluid or mixture of compressed fluids, or mixture of compressed fluids and a co-solvent, in liquid, sub-critical liquid, gaseous or supercritical state, in appropriate composition, temperature and pressure ranges;
- the contact of the resulting compressed fluid mixture and the ophthalmic articles, during an appropriate contact time, in appropriate composition, pressure and temperature ranges, and the subsequent impregnation of the drug or drug composition into the ophthalmic articles;
- the reduction of the pressure, after the contact time considered to be appropriate, and the subsequent removal of the compressed fluid or mixture of compressed fluids, or mixture of compressed fluids and a co-solvent, and the recovery of the ophthalmic articles impregnated with the drug or drug composition.

2. A method according to claim 1, **characterized in that** it comprises the following steps:
- introducing a compressed fluid or mixture of compressed fluids, or mixture of compressed fluids and a co-solvent, in a liquid, sub-critical liquid, gaseous or supercritical state, into a sealed high pressure vessel containing the ophthalmic article (or articles) and the drug (or drug composition) for impregnation. Inert and preferably non-toxic co-solvents can be added to increase the solubility of the drugs in the fluid mixture;
- retaining the compressed fluid or mixture of compressed fluids, or the mixture of compressed fluids and a co-solvent, inside the high pressure vessel under pre-established temperature and pressure operating conditions, with or without magnetic stirring, for a pre-determined period of time in order to cause the solubilization of the drug or drug composition in the compressed fluid or mixture of compressed fluids, or in the mixture of compressed fluids and a co-solvent, and impregnating the drug or drug composition in the ophthalmic article (or articles), at a pre-established depth and concentration;
- removing the compressed fluid or mixture of compressed fluids, or the mixture of compressed fluid and a co-solvent, through slow expansion from the high pressure vessel to atmospheric pressure, in order not to alter and/or damage the ophthalmic article (or articles);
- recovering the ophthalmic article, or articles, in which the drug or drug composition was impregnated at a certain depth and concentration.

3. A method according to claim 1, **characterized in that** it comprises the following steps:
- introducing a compressed fluid or mixture of compressed fluids, or mixture of compressed fluids and a co-solvent, in a liquid, sub-critical liquid, gaseous or supercritical state, into a first sealed high pressure vessel containing the drug or drug composition to be impregnated and the ophthalmic article (or articles) into a second sealed high pressure vessel;
- keeping the compressed fluid, or mixture of compressed fluids, or mixture of compressed fluid and a co-solvent, inside the first sealed vessel under pre-established operating temperature and pressure conditions, with or without magnetic stirring, for a pre-determined period of time in order to cause the solubilization of the drug or drug composition in the compressed fluid or mixture of compressed fluids, or in the mixture of compressed fluids and a co-solvent;
- transferring the resulting mixture from the first sealed vessel to the second sealed vessel, which contains the ophthalmic article or articles to be impregnated;
- retaining the mixture which contains the compressed fluid or mixture of compressed fluids, or the mixture of compressed fluids and a co-solvent, the drug or drug composition and the ophthalmic article or articles inside the second high pressure vessel under pre-established temperature and pressure operating conditions, with or without magnetic stirring, for a pre-determined period of time in order to cause the impregnation of the drug or drug composition in the ophthalmic article (or articles), at a pre-established depth and concentration;
- removing the compressed fluid or mixture of compressed fluids, or the mixture of compressed fluids and a co-solvent, through slow expansion from the second high pressure vessel to atmospheric pressure, in order not to alter and/or damage the ophthalmic article (or articles);
- recovering the ophthalmic article, or articles, in which the drug or drug composition was impregnated at a certain depth and concentration.

4. A method according to claim 1, **characterized in that** it comprises the following steps:
- introducing a compressed fluid or mixture of compressed fluids, or mixture of compressed fluids and a co-solvent, in a liquid, sub-critical liquid, gaseous or supercritical state, into a first sealed high pressure extractor containing the drug or drug composition, as a filler, and the ophthalmic article (or articles) into a second sealed high pressure vessel;
- pumping and pressurizing the compressed fluid or compressed fluids mixture, or mixture of compressed fluids and a co-solvent, into the two sealed high pressure vessels, placed in contact at a certain pre-established pressure;
- closing the connection between the two sealed high pressure vessels;
- dissolving the drug or drug composition in the compressed fluid or mixture of compressed fluids, or in the mixture of compressed fluids and a co-solvent, in the first sealed high pressure extractor, under certain pre-established temperature and pressure operating conditions, without stirring, for a pre-established period of time;
- transferring the resulting mixture from the first sealed extractor to the second sealed vessel, which contains the ophthalmic article (or articles) to be impregnated;
- pressurizing the whole system until attaining a new predefined pressure and slowly opening the system expansion valve, in order to obtain a low flow rate;
- maintaining this constant low flow rate of the compressed fluid or mixture of compressed fluids, or mixture of compressed fluids and a co-solvent, through the entire system, under pre-established temperature and pressure operating conditions, with or without magnetic stirring in the second high pressure vessel, for a pre-determined period of time in order to cause the impregnation of the drug or drug composition in the ophthalmic article (or articles), at a pre-established depth and concentration;
- completely removing the compressed fluid or mixture of compressed fluids, or the mixture of compressed fluids and a co-solvent, after the above-mentioned pre-established period of time, by means of a slow expansion of the system to atmospheric pressure, in order not to alter and/or damage the ophthalmic article (or articles);
- recovering the ophthalmic article or articles in which the drug or drug composition was impregnated at a certain depth and concentration.

5. A method according to any one of claims 1 to 4, **characterized in that** the ophthalmic article is a finished or semi-finished contact lens.

6. A method according to any one of claims 1 to 4, **characterized in that** the ophthalmic article is a finished or semi-finished intraocular lens.

7. A method according to any one of claims 1 to 4, **characterized in that** the ophthalmic article is a biocompatible and/or biodegradable solid polymer matrix or hydrogel.

8. A method according to any one of claims 1 to 4, **characterized in that** the ophthalmic articles are made of linear, branched or crosslinked polymers, copolymers or mixtures thereof, which are appropriate for manufacturing an ophthalmic article, selected from poly(acrylates), poly(hydroxyalkyl acrylates), poly(methacrylates), poly(hydroxyalkyl methacrylates), poly(alkyl acrylates), poly(alkyl methacrylates), poly(allyl methacrylates), poly(diacrylates), poly(triacrylates), poly(dimethacrylates), poly(trimethacrylates), poly(alkyl diacrylates), poly(glycerol methacrylates), poly(aminoalkyl acrylates), poly(aminoalkyl methacrylates), poly(aminoalkyl diacrylates), poly(aminoalkyl dimethacrylates), poly(ethoxybiphenol-A dimethacrylates), poly(ethyleneglycol methacrylates), poly(ethyleneglycol dimethacrylates), poly(ethyleneglycol trimethacrylates), poly(siloxanyl acrylates), poly(siloxanyl methacrylates), poly(siloxanyl alkyl acrylates), poly(siloxanyl alkyl methacrylates), poly(siloxanyl alkyl diacrylates), poly(siloxanyl alkyl dimethacrylates), poly(sylil methylene methacrilates), poly(siloxanyl alkyl vinyls), poly(siloxanyl alkyl carbamate-carbonates), poly(siloxanyl alkyl itaconates), poly(siloxanyl alkyl alkynes), poly(siloxanyl alkyl fumarates), poly(fluoro alkyl acrylates), poly(fluoro alkyl methacrylates), poly(fluoro alkyl dimethacrylates), poly(fluoro alkyl diacrylates), poly(fluoro alkyl trimethacrylates), poly(fluoro alkyl itaconates), poly(fluoro alkyl mesoconates), poly(fluoro ethers), poly(urethanes), poly(thiourethanes), poly(carbonates), poly(allyl carbonates), poly(N-vinyl pyrrolidinones), poly(diacetone acrylamides), poly(alkyl acrylamides), poly(dialkyl acrylamides), poly(hydroxyalkyl acrylamides), poly(methacrylamides), poly(acrylic acids), poly(methacrylic acids), poly(ethers), poly(esters), poly(vinyl esters), poly(orthoesters), poly(vinyl alcohols), poly(ethylene terephthalates), poly(butylene terephthalates), hydrolyzed poly(acrylonitriles), poly(siloxanes), poly(vinyl acetals), poly(phospazenes), poly(ethylene glycols), poly(propylene glycols), poly(butylene glycols), poly(ethylene oxides), poly(propylene oxides), poly(butylene oxides), poly(vinyl acetates), poly(vinyl butyrates), poly(hyaluronic acids), poly(imines), poly(ethylene imines), poly(imines), poly(amines), poly(vinyl amines), poly(caprolactones), poly(lactic acids), poly(lactic-co-glycolic acids), poly(hydroxyl alkanoates), poly(styrenes), poly(t-butyl styrenes), poly(vinyl benzenes), poly(divinylbenzenes), poly(fluorostyrenes), poly(sulfonamides), poly(sulfonates), poly (allyl sulfonates), poly(anhydrides), cellulose acetate, cellulose triacetate, cellulose acetate propionate, cellulose acetate butyrate, chitin, chitosan, modified chitosan, dextran, modified dextran, starch, modified starch, and other polymers, copolymers or mixtures thereof which can also be used for manufacturing ophthalmic articles.

9. A method according to any one of claims 1 to 4, **characterized in that** the drugs (and salts thereof), which can be impregnated in accordance with the invention and used for the treatment of eye diseases, are selected from steroid and corticosteroid anti-inflammatories (prednisolone, methylprednisolone, fluorometholone, dexamethasone, betamethasone, hydrocortisone, medrysone, loteprednol, rimexolone, triamcinolone), non-steroid anti-inflammatories and anti-allergenics (diclofenac, ketorolac, flurbiprofen, indomethacin, suprofen, ibuprofen, ketorolac tromethamine, emedastine, levocabastine, azelastine, olopatadine, ketotifen, cromolyn, lodoxamide), beta-adrenergic receptor antagonists (timolol maleate, timolol hemihydrate, metipranolol, carteolol, betaxolol, levabunolol, levobetaxolol), alpha-adrenergic receptor antagonists (brimonidine, apraclonidine), prostaglandin analogues (latanoprost, travoprost, bimatoprost), docosanoids (unoprostone isopropyl), cholinergic agonists (pilocarpine, carbachol), adenergic antagonists (epinephrine, dipivefrin, epinephryl borate), carbonic anhydrase inhibitors (dorzolamide, acetazolamide, dichlorphenamide, methazolamide, brinzolamide), vitamins and mineral supplements (vitamin A (beta-carotene), vitamin C (ascorbic acid), vitamin E (dl-alpha-tocopheryl acetate), zinc (zinc oxide), copper (cupric oxide), antibiotics (penicillins and synthetic penicillins (amoxicillin, dicloxacillin, augmentin), cephalosporins (1^{st}, 2^{nd}, and 3^{rd} generations), aminoglycosides (gentamycin, tobramycin, neomycin), macrolides (erythromycin, azithromycin, clarithromycin), fluoroquinolones (ofloxacin, ciprofloxacin, norfloxacin, levofloxacin)), antivirals (acyclovir, valacyclovir, famcyclovir), analgesics (diclofenac, acetaminophen, acetylsalicylic acid, ibuprofen, celecoxib, rofecoxib, valdecoxib, tramadol), antihistamines (diphenhydramine, chlorpheniramine, cetirizine, loratadine, desloratadine, fexofenadine), mydriatic and dilation reversal drugs (tropicamide, hydroxyamphetamine, dapiprazole), or mixtures of two or more of the above-mentioned drugs.

10. A method according to any one of claims 1 to 4, **characterized in that** the drug types which can be impregnated in accordance with the method of the present invention and used for the treatment of other human pathologies, are selected from antifungals, antibacterials, antivirals, anti-inflammatories, antibiotics, corticosteroids, antirheumatic agents, relaxants, anesthetics, analgesics, antipyretics, antiepileptics, anti-Parkinson drugs, psycholeptics, anxiolytics, hypnotic and sedative drugs, antiparasitics, antihistamines, immunoglobulins, insulins and analogues thereof, electrolytes with carbohydrates, blood glucose lowering drugs, vitamins, mineral supplements, tonics and nutrients, anabolic agents, digestives, laxatives, appetite suppressants and stimulants, antithrombotic agents, antihemorrhagic drugs, antianemic preparations, cardiac glycosides, antiarrhythmics, cardiac stimulants, vasodilators, antihypertensives, diuretics, vasoprotectives, alpha- and beta-blocking agents, calcium channel blockers, UV-radiation protectors, antiseptics, contraceptives, sex hormones and other hormonal preparations, vaccines, peptidic molecules, antineoplastic agents, aminoacids, diagnostic agents and contrast agents, or any mixtures of two or more of the above-mentioned types of drugs.

11. A method according to any one of claims 1 to 4, **characterized in that** a compressed fluid or mixture of compressed fluids are at relatively high pressures and can be in a liquid, sub-critical liquid, gaseous or supercritical state.

12. A method according to any one of claims 1 to 4, **characterized in that** the compressed fluid is selected from carbon dioxide, ethane, ethylene, propane, propylene, n-pentane, methane, trifluoromethane, chlorotrifluoromethane, trichlorofluoromethane, water, ammonia, nitrogen, nitrous oxide, ethanol, methanol, sulfur hexafluoride, cyclohexane, toluene, p-xylene, tetrafluoromethane, perfluoroethane, tetrafluoroethylene, 1,1-difluoroethylene, etc., and any mixture of two or more of the above-mentioned compressed fluids.

13. A method according to any one of claims 1 to 4, **characterized in that** the compressed fluid is carbon dioxide.

14. A method according to any one of claims 1 to 4, **characterized in that** the co-solvent is a substance selected from low molecular weight aliphatic hydrocarbons, low molecular weight aliphatic alcohols, low molecular weight aliphatic ketones, low molecular weight ethers, low molecular weight esters and low molecular weight cyclic ethers.

15. A method according to claim 4, **characterized in that** the co-solvent is selected from methanol, ethanol, 1-propanol, 2-propanol and acetone.

16. A method according to any one of claims 1 to 4, **characterized in that** the impregnation amount and penetration depth of the drug or drug composition can be varied by changing the processing conditions (temperature, pressure and length of treatment).

17. A method according to any one of claims 1 to 4, **characterized in that** the operating temperature is 30 °C or more lower than the glass transition temperature under normal conditions of the polymers or copolymers which compose the ophthalmic article processed and can be changed or kept constant throughout the process.

18. A method according to claim 17,
**characterized in that** the operating temperature is about 50 °C lower than the glass transition temperature.

19. A method according to any one of claims 1 to 4, **characterized in that** the operating temperature is between 30 °C and 100 °C and can be changed or kept constant throughout the process.

20. A method according to any one of claims 1 to 4, **characterized in that** the operating pressure is between 30 bar and 400 bar, being able to dissolve the drug or drug composition to such an extent that it is possible to carry out a subsequent impregnation at the desired therapeutic concentration levels, and can be changed or kept constant throughout the process.

21. A method according to claim 20,
**characterized in that** the operating pressure is between 60 bar and 250 bar and can be changed or kept constant throughout the process.

22. A method according to any one of claims 1 to 4, **characterized in that** the length of treatment is between 30 and 360 minutes, mainly depending on the desired drug or drug composition impregnation depth, amount of drug or drug composition to be impregnated into the ophthalmic articles to be processed, the nature of the polymers and copolymers, compressed fluids (or mixture of compressed fluids), co-solvents and drugs (or drug composition) involved in the process, the specific interactions that may occur between all the contacted materials, the specific operating conditions applied and other important characteristics of the ophthalmic articles, such as the type of use (daily, monthly, bimonthly) dimension and geometry.

23. A method according to any one of claims 1 to 4, **characterized in that** the length of treatment is between 30 and 180 minutes.
